# NEW EUROPEAN PATENT SPECIFICATION

(11) **EP 1 814 480 B2**
(45) Date of publication and mention of the opposition decision: **22.02.2017**
(45) Mention of the grant of the patent: 02.09.2009
(21) Application number: 05851833.3
(22) Date of filing: 18.11.2005
(51) Int. Cl.: A61F 2/78, B29C 33/40, B28B 1/00

(54) **NOVEL ENHANCED COATING FOR PROSTHETIC LINERS PROCESSES, PRODUCTS AND IMPROVED UMBRELLAS**
NEUE VERBESSERTE BESCHICHTUNG FÜR PROTHETISCHE EINSATZPROZESSE, PRODUKTE UND VERBESSERTE SCHIRME
REVETEMENT AMELIORE POUR CHEMISAGE DE PROTHESES, PRODUITS ET PARAPLUIES AMELIORES

(30) Priority: 22.11.2004 US 630108 P; 15.03.2005 US 662182 P; 17.06.2005 US 156028
(43) Date of publication of application: 08.08.2007
(73) Proprietor: Freedom Innovations, LLC, Irvine CA 92618-2012 (US)
(72) Inventor: SHANNON, Donald T., Trabuco Canyon, California 92679 (US)
(74) Representative: Crease, Devanand John
(86) International application number: PCT/US2005/041890
(87) International publication number: WO 2006/057916

(56) References cited:
- WO-A-01/67842
- WO-A-03/051241
- US-A- 4 908 037
- US-A- 5 746 772
- US-A- 5 830 237
- US-B1- 6 231 617
- US-B1- 6 454 812

## Description

### BACKGROUND

The present disclosure relates to technologies for amputees utilized with prosthetics and prosthetic coatings to make ease of use and related issues more easily managed. An example of a prior art liner for a prosthetic device is given in document WO 01/67 842. Particularly, the present disclosure relates to lubricious coating on linings and prosthetics used for amputees inside, outside, and throughout the liners and prosthetics. An example of a lubricated liner is disclosed in document WO 03/051 241.

Longstanding needs in the art are related to the fact that the residuary limb of an amputee undergoes structural changes over time, and those who wish to engage in active lifestyles subject their respective prostheses to untoward stresses over time.

Typical amputees' residua tend to decrease in mass over time, owing in part to atrophy, and other stresses to which they are generally subjected. For this reason the teachings of the present disclosure are offered for consideration and believed to provide an improved way for amputees to lead active lifestyles without any compromise to the fit, comfort, or durability of their respective liner systems.

The prior art has turned to adding supplemental structural elements to prosthetics and prosthetic liners, which impacts ease of use, comfort, and other aspects which users do not enjoy, especially those engaged in active lifestyles. Accordingly, by addressing, ameliorating, and overcoming these longstanding issues, the teachings of the instant disclosure are believed to constitute progress in the science and the useful art susceptible of a Letter Patent grant, and such relief is hereby earnestly solicited.

### Brief Description of the Figures

Fig. 1 is a schematic of a liner according to the present disclosure;
Fig. 2 is a schematic of a liner according to the present disclosure; and,
Fig. 3 is a schematic of the distal end of a liner according to the present disclosure.

### Summary

There is provided a prosthetic medical device, comprising a prosthetic liner comprising at least one layer of a protective and lubricious coating being coated on the periphery of the liner whereby owing to the lowered coefficient of friction, the liner may be donned and doffed by physically challenged users, or by a user with only one hand; and, an enhanced umbrella attachment reduces pistoning between the bottom of the liner and re-inforced sections adjacent to and above the same, wherein the prosthetic further comprises at least one layer of coating containing Parylene (poly-paraxylylene) whereby the prosthetic liner is coated over substantially all of its surface area.

The Parylene may be selected from the group consisting of Parylene N (poly-para-xylylene), Parylene D (poly-dichloro-para-xylylene), Parylene C (poly-monochloro-para-xylylene ) and Parylen HT.

The coating may be vapor deposited on the liner.

There is also provided process for creating a prosthetic liner having an enhanced coating, comprising the steps of plugging a female mold; inserting a pin umbrella; covering a male mold with a cloth; saturating the cloth for integration into the resulting product; adding at least one aliquot of liquified silicone; inserting a male mold member into the female mold member; heating the assembled article; and coating at least one layer of a protective and lubricious coating on the periphery of the liner, wherein, the coating is charaterized in that it consists essentially of at least one Parylene selected from the group consisting of: Parylene N (poly-para-xylylene), Parylene D (poly-dichloro-para-xylylene), Parylene C (poly-monochloro-para-xylylene ), Parylen HT and combinations thereof whereby the prosthetic liner is coated over substantially all of its surface area.

The process may be done under vacuum pressure during a time interval of less than an hour.

Outgassing may be the mechanism which the deposition of the coating layer is facilitated.

Deposition of complex molecular coatings are done according to known processes using known means for depositing them on surfaces as thing films or coatings. This is done to combat excessive wear and high stress on these surfaces, according to the present disclosure, prosthetics and prosthetic liners are shown, which improve resistance to stresses during normal and enhanced stress, for example, exercise processes for amputees.

### DETAILED DESCRIPTION

The present inventor has discovered that laminated materials preventing the types of failures can be simply and elegantly generated to address the longstanding needs existing among the prior art. The laminates of the instant teachings include more than one layer of coated or uncoated liner or prosthetic material.

Those skilled in the art will understand that the prosthetic or prosthetic liner has a lubricous coating on the outside and inside. Artisans will know, the coating is Parylene from Para Tech of Aliso Viejo. The coating can be vapor deposited.

What is taught is a process for attaching at least a lubricious coating layer. The prosthetic or prosthetic liner has a lubricous coating on the outside and inside, and wherein the coating is vapor deposited, or otherwise applied.

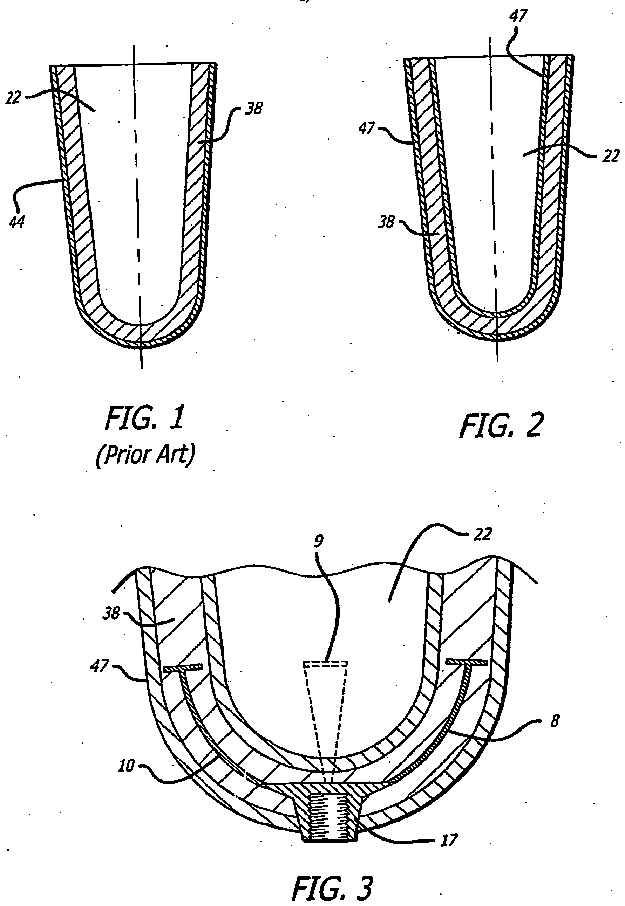

Turning to Fig. 1 liners for prosthetics 38 often fit over the residuum 22 with a coating 40 applied on the outside of the prosthetic to allow it to be put on and off.

Fig. 2 shows an improved coating 47 over liner 38 covering residuum 22. With the instant coating, users who are physically challenged can doff and don the coating without having to make great physical efforts to do so.

Likewise, those who are physically fit and strong enough to use one hand may do so without exertion that is excessive. This is a substantial improvement.

Turning to Fig. 3, a novel pin umbrella 17 (which attaches to a prosthetic) is disclosed whereby side walls 10, 9 and 8 extend to a point where a cloth reinforcement layer begins to reduce piston motioning. Liner 38 and coating 47 are also shown.

What is unexpectedly better than dictated by empirical data are the excellent conformability and sealing properties, including the self-attachment achieved with the instant prosthetics and prosthetic linings generally for amputees.

Moreover, such coating layers allow for comfortable liners in high stress situations. Prior liners often required the wearer to opt for either high durability and low comfort, or less durable, high comfort liners. The instant disclosure solves these problems because coatings, in addition to providing a dry lubricant, also serve as a protectant, allowing for high stress without the need to sacrifice comfort.

The contemplated coatings are versatile. A coating can be applied to the entire surface of a liner or prosthetic.

In the present disclosure, Parylene is applied to a prosthetic liner as a dry lubricant and protective coating. Thus, the entire liner could be coated with Parylene (available from Para Tech Coating, Inc., Aliso Viejo, California, 92656) except for gripping zones that would be used to pull the liner of the residuum of an appendage. Thus, the liner would slide easily over the skin due to the coating, but also allow the amputee to firmly grasp the liner in order to pull it over the residuum of the former appendage.

Parylene is a vacuum deposited film originating from a powder (di-para-xylylene). The powder is converted to a gaseous monomer that condenses and polymerizes on substrates at room temperature. Unlike dip or spray coatings, condensation coating does not run off or sag, is pinhole free, and will not bridge. Thus, Parylene coats holes evenly, as well as coats over edges and internal areas.

Parylene begins as a dimer. The process is a relatively simple vacuum application system, in which the dimer is placed in the vacuum system and converted to a reactive monomer vapor. When passed over room temperature objects, this vapor rapidly coats them by polymerizing on their surfaces. The end result is an almost impervious uniform coating. Moreover, the small static and dynamic friction coefficients allow polymerized Parylene to serve as a "dry film" lubricant.

Parylene is not produced or sold as a polymer. It is not practical to melt, extrude, mold, or calendar as with thermoplastics. Nor can it be applied from solvent systems. Actually, many of the advantages found in Parylene coatings, unlike epoxies, urethanes, or silicones are a direct result of the coating deposition process. There are no environmental concerns by using Parylene because application requires no catalysts or solvents.

Parylene is extremely resistant to chemical attack, exceptionally low in trace metal contamination, with superior dielectric strength, low dissipation factor, and other superior electrical properties that remain virtually constant with changes in temperature (please refer to the Galxyl® specification sheet available at www.parylene.com). In current commercial applications, Parylene is deposited in thickness ranging from a few thousand angstroms to about 50 microns depending on the function the Parylene film must perform. A Parylene coating provides physical barrier properties equal to or better than 0,05 mm - 0,15 mm (2-6 mil) thickness applications of epoxies, silicones, urethanes, or other conventional coatings, which generally require multiple applications for accurate protection. This leads to more bulky and uneven applications.

Parylene C is poly-monochloro-para-xylylene and is the most widely used member of this unique polymer series due to its excellent barrier properties. It offers significantly lower permeability to moisture and gases, such as nitrogen, oxygen, carbon dioxide, hydrogen sulfide, sulfur dioxide and chlorine, while retaining excellent electrical properties. Other versions commercial versions of Parylene contemplated in the instant disclosure are Parylene N (poly-para-xylylene), Parylene D (poly-dichloro-para-xylylene) and Parylene HT.

While the apparatus and method have been described in terms of what are presently considered to be the most practical and preferred embodiments, it is to be understood that the disclosure need not be limited to the disclosed embodiments. It is intended to cover various modifications and similar arrangements included within the scope of the claims, the scope of which should be accorded the broadest interpretation so as to encompass all such modifications and similar structures. The present disclosure includes any and all embodiments of the following claims.

## Claims

1. A prosthetic medical device, comprising:
a prosthetic liner comprising an umbrella attachment, **characterised in that** said liner comprising at least one layer (47) of a protective and lubricious coating being coated on the periphery of the liner (38) whereby owing to the lowered coefficient of friction, the liner may be donned and doffed by physically challenged users, or by a user with only one hand; and said umbrella attachment is an enhanced umbrella (17, 8, 9, 10) attachment which reduces pistoning between the bottom of the liner and re-inforced sections adjacent to and above the same, wherein the prosthetic further comprises at least one layer of coating (47) containing Parylene whereby the prosthetic liner (38) is coated over substantially all of its surface area.

2. The prosthetic medical device of claim 1 wherein the Parylene is selected from the group consisting of Parylene N, Parylene D, Parylene C and Parylen HT.

3. The improved prosthetic of claim 1 or 2 wherein the coating (47) is vapor deposited on the liner.

4. A process for creating a prosthetic liner (38) having an enhanced coating, comprising the steps of:
plugging a female mold; inserting a pin umbrella (17, 8, 9, 10); covering a male mold with a cloth; saturating the cloth for integration into the resulting product; adding at least one aliquot of liquified silicone; inserting a male mold member into the female mold member; heating the assembled article; and coating at least one layer of a protective and lubricious coating (47) on the periphery of the liner (38), wherein, the coating is **characterized in that** it consists essentially of at least one Parylene selected from the group consisting of: Parylene N, Parylene D, Parylene C, Parylen HT and combinations thereof whereby the prosthetic liner (38) is coated over substantially all of its surface area.

5. The method of claim 4, whereby the process is done under vacuum pressure during a time interval of less than an hour.

6. The method of claim 5, whereby outgassing is the mechanism which the deposition of the coating layer is facilitated.

## Patentansprüche

1. Medizinische Prothese, die Folgendes umfasst:
Eine Prothesenauskleidung mit einer Schirmbefestigung, **dadurch gekennzeichnet, dass** diese Auskleidung zumindest eine Schicht (47) aus einem schützenden und gleitfähigen Überzug, der auf den Umfang der Auskleidung (38) aufgebracht wurde, umfasst, wobei die Auskleidung aufgrund des erniedrigten Reibungskoeffizienten von Anwendern mit einer körperlichen Behinderung oder von einem Anwender mit nur einer Hand an- und ausgezogen werden kann und worin die Schirmbefestigung eine verbesserte Schirmbefestigung (17, 8, 9, 10) ist, die eine Kolbenbewegung zwischen der Unterseite der Auskleidung und verstärkten Abschnitten neben und über dieser verringert, worin die Prothese ferner zumindest eine Überzugsschicht (47) aus Parylen umfasst worin die Prothesenauskleidung (38) im Wesentlichen über ihrer gesamten Oberfläche beschichtet ist.

2. Medizinische Prothese nach Anspruch 1, worin das Parylen aus der aus Parylen N, Parylen D, Parylen C und Parylen HT bestehenden Gruppe ausgewählt ist.

3. Verbesserte Prothese nach Anspruch 1 oder 2, worin der Überzug (47) auf die Auskleidung dampfabgeschieden wird.

4. Verfahren zur Herstellung einer Prothesenauskleidung (38) mit einem verbesserten Überzug, das folgende Schritte umfasst:
Ausgießen einer Negativform; Einführen eines Stiftschirms (17, 8, 9, 10); Abdecken einer Positivform mit einem Tuch; Sättigen des Tuchs zur Integration in das resultierende Produkt; Zufügen zumindest eines Aliquots von verflüssigtem Silikon; Einführen eines Positivformelements in das Negativformelement; Erwärmen des zusammengesetzten Artikels; und Beschichten mit zumindest einer Schicht eines schützenden und gleitfähigen Überzugs (47) auf dem Umfang der Auskleidung (38), worin der Überzug **dadurch gekennzeichnet ist, dass** er im Wesentlichen aus zumindest einem Parylen besteht, das aus der aus Parylen N, Parylen D, Parylen C, Parylen HT und Kombinationen davon bestehenden Gruppe ausgewählt ist worin die Prothesenauskleidung (38) im Wesentlichen über ihrer gesamten Oberfläche beschichtet ist.

5. Verfahren nach Anspruch 4, worin das Verfahren während eines Zeitraums von weniger als einer Stunde unter Vakuumdruck durchgeführt wird.

6. Verfahren nach Anspruch 5, worin der Mechanismus, mit dem die Abscheidung der Überzugsschicht erleichtert wird, Ausgasen ist.

## Revendications

1. Dispositif médical prothétique, comprenant :
une chemise prothétique comprenant une attache en forme de parapluie, **caractérisé en ce que** ladite chemise comprend au moins une couche ( 4 7) en un revêtement protecteur et lubrifiant appliqué sur la périphérie de la chemise (38), la chemise pouvant être mise en place et enlevée par des utilisateurs physiquement handicapés, ou par un utilisateur n'ayant qu'une seule main, en raison de son coefficient de friction réduit et ladite attache en forme de parapluie étant une attache améliorée en forme de parapluie (17, 8, 9, 10) qui réduit l'effet de piston entre le fond de la chemise et les sections renforcées adjacentes à celle-ci et au-dessus de celle-ci, la prothèse comprenant en outre au moins une couche de revêtement (47) contenant du Parylène dans laquelle la chemise prothétique (38) est revêtue substantiellement sur toute sa superficie.

2. Dispositif médical prothétique selon la revendication 1, dans lequel le Parylène est choisi parmi le groupe constitué du Parylène N, du Parylène D, du Parylène Cet du Parylène HT.

3. Prothèse améliorée selon les revendications 1 ou 2, dans laquelle le revêtement (47) est déposé en phase vapeur sur la chemise.

4. Procédé pour créer une chemise prothétique (38) ayant un revêtement amélioré, comprenant les étapes suivantes :
brancher un moule femelle ; insérer un parapluie mâle (17, 8, 9, 10) ; couvrir un moule mâle avec un tissue ; saturer le tissu pour l'intégrer dans le produit résultant ; ajouter au moins une aliquote de silicone liquéfiée ; insérer un organe de moule mâle dans l'organe de moule femelle ; chauffer l'article assemblé ; et appliquer au moins une couche d'un revêtement protecteur lubrifiant (47) sur la périphérie de la chemise (38), le revêtement étant **caractérisé en ce qu'**il consiste essentiellement en au moins un Parylène choisi parmi le groupe constitué du Parylène N, du Parylène D, du Parylène C, du Parylène HT et de leurs combinaisons dans laquelle la chemise prothétique (38) est revêtue substantiellement sur toute sa superficie.

5. Procédé selon les revendication 4, dans lequel le processus est effectué sous une pression de vide au cours d'un intervalle de temps inférieur à une heure.

6. Procédé selon la revendication 5, dans lequel l'évacuation du gaz est le mécanisme par le biais duquel le dépôt de la couche de revêtement est facilité.
